# EUROPEAN PATENT SPECIFICATION

(11) **EP 2 404 903 B1**
(45) Date of publication and mention of the grant of the patent: **17.07.2013**
(21) Application number: 10748713.4
(22) Date of filing: 01.03.2010
(51) Int. Cl.: C07D 207/404, A61K 51/00, C07B 59/00, C07B 61/00

(54) **METHOD OF SYNTHESIZING [18F]SFB USING MICROSYNTHESIS TECHNIQUE**
VERFAHREN ZUR SYNTHESE VON [18F]SFB MITHILFE EINER MIKROSYNTHESETECHNIK
MÉTHODE DE SYNTHÈSE DU [18F]SFB PAR UNE TECHNIQUE DE MICROSYNTHÈSE& xA;

(30) Priority: 04.03.2009 JP 2009050814
(43) Date of publication of application: 11.01.2012
(73) Proprietor: Shimadzu Corporation, Kyoto-shi, Kyoto 604-8511 (JP); Kyoto University, Sakyo-ku Kyoto-shi Kyoto 606-8501 (JP)
(72) Inventor: NAKANISHI Hiroaki, Kyoto-shi Kyoto 604-8511 (JP); SAIKI Hidekazu, Kyoto-shi Kyoto 604-8511 (JP); SAJI Hideo, Kyoto-shi Kyoto 606-8501 (JP); KIMURA Hiroyuki, Kyoto-shi Kyoto 606-8501 (JP); KAWASHIMA Hidekazu, Kyoto-shi Kyoto 606-8501 (JP); TOMATSU Kenji, Kyoto-shi Kyoto 606-8501 (JP); KUGE Yuji, Kyoto-shi Kyoto 606-8501 (JP)
(74) Representative: Müller-Boré & Partner Patentanwälte
(86) International application number: PCT/JP2010/053252
(87) International publication number: WO 2010/101118

(56) References cited:
- JP-A- 2004 313 867
- JP-A- 2008 037 767
- TANG, G. ET AL.: 'Facile synthesis of N-succinimidyl 4- [18F] fluorobenzoate ([18F] SFB) for protein labeling' JOURNAL OF LABELLED COMPOUNDS AND RADIOPHARMACEUTICALS vol. 51, 2008, pages 68 - 71
- MILLER, P.W. ET AL.: 'Radiolabelling with short-lived PET (positron emission tomography) isotopes using microfluidic reactors' J CHEM TECHNOL BIOTECHNOL vol. 84, no. 3, 30 October 2008, pages 309 - 315
- BANNWARTH, W. ET AL.: 'Formation of carboxamides with N, N, N', N'-tetramethyl (succinimido) uronium tetrafluoroborate in aqueous/ organic solvent systems' TETRAHEDRON LETTERS vol. 32, no. 9, 1991, pages 1157 - 1160
- 'Abstracts of Annual Meeting of Pharmaceutic', vol. 129, 05 March 2009 article KENJI TOMATSU ET AL.: 'Microreactor o Mochiita N-succinimidyl 4-[18F]fluorobenzoate ([18F]SFB) no Jinsoku Gosei Ho no Kaihatsu', page 86

## Description

### Technical Field

The present invention relates to a method for synthesizing [¹⁸F]SFB (N-succinimidyl 4-[¹⁸F]fluorobenzoate) for use as a reagent for labeling high-molecular weight compounds such as peptides and antibodies or low-molecular weight compounds with [¹⁸F], which is an RI (radioisotope), to synthesize PET (Positron Emission Tomography) diagnostic probes.

### Background Art

In PET diagnosis, ¹⁸F which is an RI with a short half-life of about 110 minutes is often used. Since ¹⁸F has such a short half-life, ¹⁸F-labeled diagnostic probes are produced using an on-site automatic synthesizing apparatus. The current most useful PET diagnostic probe is ¹⁸F-labeled fluorodeoxyglucose (¹⁸F-FDG) useful for early detection of cancer. Since ¹⁸F-FDG became covered by health insurance, the number of private PET centers has been rapidly increased to over 100. It is believed that this reflects great expectations of society on early detection of cancer. Therefore, it can be said that PET is an advanced diagnostic technique but is becoming a common diagnostic technique rooted in society. In order to respond to such social demands and further develop PET diagnosis, it is necessary to establish a technique for automatically synthesizing PET diagnostic probes. Without it, it is impossible to expect that PET diagnosis will become common and contribute to society.

An example of a labeling reagent for use in synthesizing PET diagnostic probes other than ¹⁸F-FDG is [¹⁸F]SFB. [¹⁸F]SFB is widely used as a reagent for labeling peptides, antibodies, and polymers.

As a method for synthesizing [¹⁸F]SFB, a batch synthesis method using a three-step reaction is known. As such a batch synthesis method, a one-pot procedure has been reported (see Non-Patent Document 1), which is a modification of a two-pot procedure using two reactors. The one-pot procedure is a method in which reaction reagents are added to one reactor in order. According to the one-pot procedure, in a first-step reaction, ethyl 4-(trimethylammonium triflate) benzoate as a labeled precursor is fluorinated with [¹⁸F] to obtain ethyl 4-[¹⁸F]fluorobenzoate. Then, in a second-step reaction, ethyl 4-[¹⁸F]fluorobenzoate is saponified with a tetrapropylammonium hydroxide solution to obtain a 4-[¹⁸F]fluorobenzoic acid salt ([¹⁸F]FBA) as a [¹⁸F]SFB intermediate. Then, in a third-step reaction, the intermediate is reacted with
O-(N-succinimidyl)-N,N,N' ,N' -tetramethyluronium tetrafluoroborate to obtain target [¹⁸F]SFB (see Non-Patent Document 1).

In such a series of reactions, [¹⁸F]FBA, which is a product of the second-step reaction and a [¹⁸F]SFB intermediate, is purified by solid-phase extraction and azeotropically dehydrated using acetonitrile (MeCN) before the start of the third-step reaction.
Prior Art Documents
Non-Patent Documents

Non-Patent Document 1: G. Tang et al., J. Label. Compd. Radiopharm., 51, 68-71 (2008)
Non-Patent Document 2: F. Wust et al., Appl. Radiat Isot., 59, 43-48 (2003)
Non-Patent Document 3: HJ. Wester et al., Nucl. Med. Biol., 23, 365-372 (1996)
Non-Patent Document 4: W. Bannwarth et al., Tetrahedron Letters 32, 1157-1160 (1991)

### Disclosure of the Invention

### Problem to be Solved by the Invention

In addition to the above-described method, there are other methods for synthesizing [¹⁸F]SFB. However, all these methods are batch synthesis methods that are common in that reaction reagents are added to one or two reactors in order. These synthesis methods take a long time of 1 to 1.5 hours to synthesize [¹⁸F]SFB and require complicated operations, and therefore under the present circumstances, synthesis of [¹⁸F]SFB is performed in only part of PET centers and the like.

In the case of manual synthesis, operations are complicated and, therefore, advanced skills are required. On the other hand, apparatuses that can automatically synthesize [¹⁸F]SFB are commercially available from overseas, but are large in size and expensive. Therefore, PET centers and the like that can introduce such apparatuses are limited.

In order to solve the above problems, synthesis of [¹⁸F]SFB needs to be improved from the following viewpoints:
(1) to improve reaction efficiency, that is, to improve radiochemical yield and to reduce synthesis time;
(2) to simplify operation techniques; and
(3) to reduce production costs for pharmaceutical synthesis.

It is therefore an object of the present invention to reduce the time of [¹⁸F]SFB synthesis by improving reaction efficiency, simplify operations for [¹⁸F]SFB synthesis, and reduce the production cost of [¹⁸F]SFB.

### Means for Solving the Problem

Basically, the amounts of solutions used in a reaction using a radioactive substance are very small, and therefore, there are limitations to conventional batch synthesis methods including one-pot synthesis methods. On the other hand, the present invention uses microprocessing technology that has rapidly advanced in recent years to perform a reaction by allowing a reaction solution to flow through a microchannel provided in a microreactor.

The present invention is directed to a method for synthesizing [¹⁸F]SFB comprising using a microreactor including a substrate with one channel formed therein so as to have a cross-sectional width of 1 mm or less and a cross-sectional depth of 1 mm or less, the channel being connected to a raw material injection port and a first reagent injection port at one end thereof, and connected to a second reagent injection port at a position far from the one end thereof, and connected to a third reagent injection port at a position far from the second reagent injection port in a direction toward another end thereof, and connected to a liquid discharge port at the other end thereof; and continuously performing the following three-step reaction by allowing a reaction solution to flow through the channel serving as a reaction channel from the one end to the other end thereof without taking out the reaction solution from anywhere between the one end and the other end of the channel:
a first-step reaction in which a solution of a labeled precursor of [¹⁸F]SFB and a solution containing ¹⁸F ions as a first reagent are continuously supplied from the raw material injection port and the first reagent injection port, respectively to fluorinate the labeled precursor in the channel between the one end and the second reagent injection port;
a second-step reaction in which a TPAH (tetrapropylammonium hydroxide) solution as a second reagent is continuously supplied from the second reagent injection port to saponify a reaction product of the first-step reaction in the channel between the second reagent injection port and the third reagent injection port so that a [¹⁸F]SFB intermediate is produced; and
a third-step reaction in which a TSTU (O-(N-succinimidyl)-N,N,N' ,N' tetramethyluronium tetrafluoroborate) solution as a third reagent is continuously supplied from the third reagent injection port to produce [¹⁸F]SFB in the channel between the third reagent injection port and the other end.

The method according to the present invention is a one-flow synthesis method in which a reaction from the beginning of the first-step reaction to the end of the third-step reaction is performed in one channel provided in the microreactor without taking out a reaction solution from anywhere between the one end and the other end of the channel to perform some kind of treatment on the reaction solution. For the purpose of setting reaction conditions or verifying that the reaction has proceeded properly, a reaction solution is taken out from the channel after the completion of the first-step reaction or after the completion of the second-step reaction to measure a reaction product However, this is performed for pretreatment or verification of the present invention, and is therefore not the present invention itself. Therefore, according to the present invention, the intermediate produced by the second-step reaction is neither purified nor dehydrated before the beginning of the third-step reaction.

Non-Patent Document 1 does not describe whether the third-step reaction proceeds without performing such purification and dehydration processes. On the other hand, it has been reported that TSTU used in the third-step reaction reacts with a carboxyl group even when water is present in a reaction solution (see Non-Patent Document 4). However, it is impossible to tell from the description of Non-Patent Document 4 whether the third-step reaction proceeds in a reaction system according to the present invention with such reaction efficiency that improvement in radiochemical yield and reduction in synthesis time can be achieved.

The present inventors have found that, even when such purification and dehydration processes are not performed, the third-step reaction proceeds with such reaction efficiency that the original object of the present invention can be achieved. Based on the finding, the present invention provides, for the first time, a [¹⁸F]SFB synthesis method in which the series of reactions is continuously performed in one channel.

### Effect of theInvention

According to the present invention, it is possible to significantly reduce synthesis time by introducing microsynthesis techniques, whereas conventional methods require 1 to 1.5 hours for synthesis. This is because the reaction efficiency is improved by performing a reaction in a microspace provided in a microreactor so that the reaction is completed in a short period of time. Further, steps after preparation of reagents are automatically performed by continuous solution sending, and therefore, the reproducibility of synthesis is easily achieved. The reaction device itself can be easily operated and does not require advanced skills. Further, the reaction can be performed with small amounts of reagents and solvents, and therefore, production costs for pharmaceutical synthesis can be reduced.

More specifically, conditions for one-flow synthesis of [¹⁸F]SFB were examined using a microchip for three-step reaction, and as a result, [¹⁸F]SFB was obtained in a decay-corrected radiochemical yield of about 60% in a synthesis time of 15 minutes. As can be seen from the result, the present invention makes it possible to achieve a higher yield and a shorter synthesis time as compared to conventional synthesis methods. Further, synthesis after preparation of reagents is automatically performed, which is advantageous in that reproducibility of experiments is easily achieved by anyone, the reaction can be performed with small amounts of reagents and solvents, and synthesis of [¹⁸F]SFB can be performed in a small space. Such a system has a wide range of applications from clinical use to laboratory use for PET pharmaceutical synthesis.

### Brief Description of the Drawings

Fig. 1 shows a reaction formula representing a [¹⁸F]SFB synthesis method used in an embodiment of the present invention.
Fig. 2 is a graph showing the results of an experiment to determine whether a dehydration process is required to allow a third-step reaction to proceed in the embodiment of the present invention.
Fig. 3 is a plan view of a microchip for analyzing a reaction product of a first-step reaction.
Fig. 4 is a schematic perspective view of a temperature regulation system for regulating the temperature of a microreactor used in the embodiment of the present invention.
Fig. 5 is a graph showing the results of examining the influence of temperature on the first-step reaction with the use of the microchip shown in Fig. 3.
Fig. 6 is a graph showing the results of comparison of radiochemical yield between when a second-step reaction is performed in the microreactor and when it is performed in a conventional reactor.
Fig. 7 is a graph showing the results of comparison of radiochemical yield when different solvents are used in the embodiment of the present invention.
Fig. 8 is a plan view of a microchip used as a microreactor for implementing the embodiment of the present invention.
Fig. 9 is a liquid chromatogram of a reaction product obtained in the embodiment of the present invention.

### Mode for Carrying out the Invention

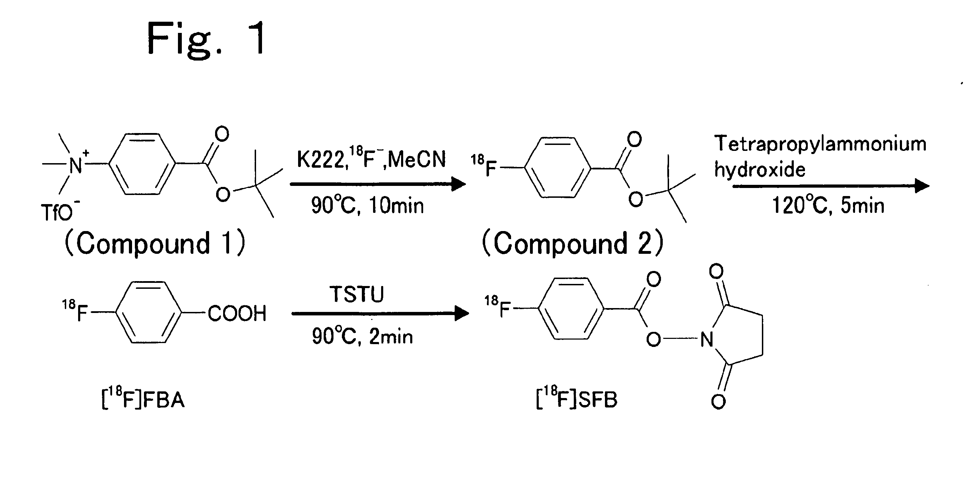

A reaction mechanism of synthesizing [¹⁸F]SFB used in an embodiment of the present invention is shown in Fig. 1.
In a first-step reaction, t-butyl 4-N,N,N-trimethyl-ammoniumbenzoate triflate (compound 1) was used as a labeled precursor. The compound 1 was synthesized using special grade reagents manufactured by Merck and Sigma Aldrich by a method described in Non-Patent Document 2 or 3. As a first reagent, K222/K[¹⁸F]F complex was used, which was obtained by eluting ¹⁸F produced by a cyclotron into a mixed solution of K₂CO₃ and Kryptofix (registered trademark) 222 (4,7,13,16,21,24-Hexaoxa-1,10-diazabicyclo[8.8.8]-hexacosane; hereinafter referred to as "K222"), drying the mixed solution to obtain a dried product, and subjecting the dried product to azeotropic dehydration using acetonitrile.

The compound 1 and the first reagent are separately supplied as solutions to two liquid injection ports of a microreactor. As a solvent of these solutions, acetonitrile or DMSO was used. As a solvent of reaction reagents used in a second-step reaction and a third-step reaction, the same solvent as used in the first-step reaction was used.

As a product of the first-step reaction, t-butyl 4-[¹⁸F]fluorobenzoate (compound 2) is produced.

In the second-step reaction, a TPAH (tetrapropylammonium hydroxide) solution is continuously supplied as a second reagent to a reaction solution containing the compound 2 as a product of the first-step reaction through a second reagent injection port As a result of a reaction between the compound 2 and TPAH, the compound 2 is saponified so that [¹⁸F]FBA (4-[¹⁸F]fluorobenzoic acid salt) as a [¹⁸F]SFB intermediate is produced.

In the third-step reaction, a TSTU (O-(N-succinimidyl)-N,N,N' ,N' -tetramethyluronium tetrafluoroborate) solution is continuously supplied as a third reagent to a reaction solution containing the [¹⁸F]SFB intermediate as a product of the second-step reaction through a third reagent injection port As a result, [¹⁸F]SFB is produced between the third reagent injection port and the other end of a channel of the microreactor.

As the labeled precursor, an ester (e.g., ethyl ester, t-butyl ester, pentamethyl ester) of 4-(trimethylammoniumtrifate)benzoic acid can be used. The compound 1 described above is a t-butyl ester of 4-(trimethylammoniumtrifate)benzoic acid.

Synthesis of [¹⁸F]SFB performed by such a three-step reaction mechanism as described above requires complicated experimental procedures, and therefore, conventional synthesis methods require 1 to 1.5 hours for synthesis. For the purpose of achieving easy and rapid synthesis, the present invention uses a microreactor that achieves reaction efficiency higher than that of conventional macro-scale synthesis.

Conventional synthesis methods require purification of the intermediate [¹⁸F]FBA by solid-phase extraction and azeotropic dehydration using acetonitrile. Therefore, it is difficult for such conventional synthesis methods to achieve one-flow synthesis while performing such purification and dehydration processes in the course of the series of reactions. On the other hand, according to the present invention, target [¹⁸F]SFB can be synthesized without performing such purification and dehydration processes in the course of the series of reactions. The reason for this will be described below.

In reference to the [¹⁸F]SFB synthesis method described in Non-Patent Document 1, the third-step reaction was performed without performing purification and dehydration of a product of the second-step reaction. This experiment was performed using a conventional reactor. More specifically, Kryptofix 222 (K222, 10 mg) was dissolved in acetonitrile (500 µL), and ¹⁸F (100 to 500 µL of a 33 mM aqueous K₂CO₃ solution containing ¹⁸F⁻ dissolved therein) was added thereto. The mixture was azeotropically dehydrated at 110°C under the stream of argon gas, and was further azeotropically dehydrated by adding acetonitrile (400 µL). Azeotropic dehydration using acetonitrile (400 µL) was repeated three times to completely remove water. Then, the compound 1 (as a 5 mg/1 mL acetonitrile solution) was added thereto to perform a reaction at 90°C for 10 minutes. Then, 20 µL of a 1 M aqueous TPAH solution was added to perform a reaction at 120°C for 5 minutes to synthesize an intermediate [¹⁸F]FBA. After synthesis, a reaction solution was azeotropically dehydrated with acetonitrile and then reacted with TSTU to synthesize [¹⁸F]SFB. On the other hand, a reaction solution whose water content was adjusted to 1.6 to 51% was reacted with TSTU without performing dehydration to synthesize [¹⁸F]SFB.

The [¹⁸F]SFB was fractionated by high-performance liquid chromatography (HPLC), and comparison of the yield of [¹⁸F]SFB was made. The fractionation by HPLC was performed using a solution of water/MeCN = 60/40 as a mobile phase at a flow rate of 1 mL/min. The radioactivity of ¹⁸F was measured using a Curie meter or a NaI (Tl) gamma scintillation counter.

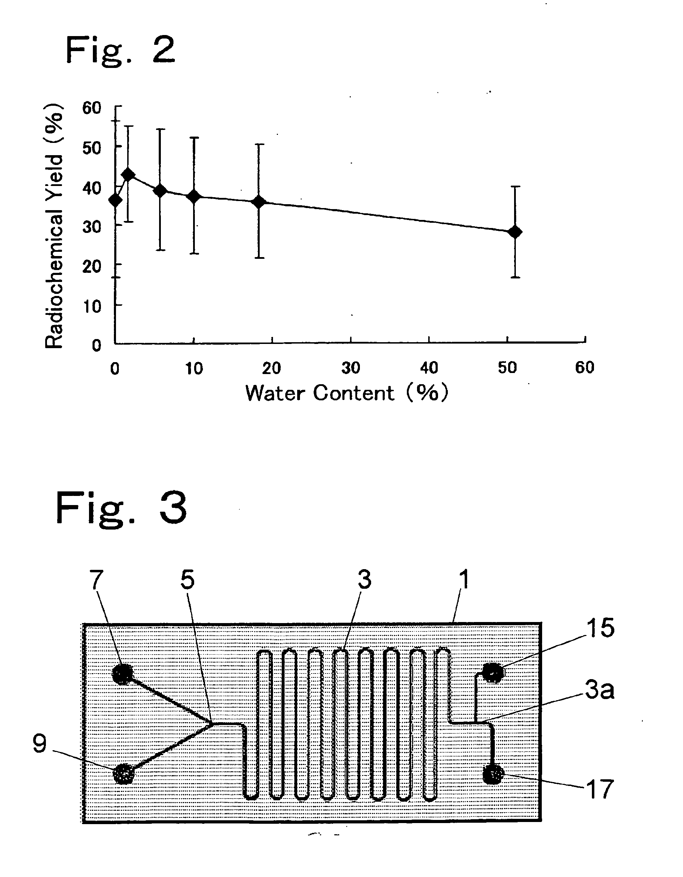

The measurement results are shown in Fig. 2. In Fig. 2, the vertical axis represents a decay-corrected radiochemical yield. As can be seen from the results shown in Fig. 2, the radiochemical yield is not greatly influenced by the water content of the reaction solution as long as the water content is low (about 20% or less). TPAH used as a reaction reagent in the second-step reaction is in the form of a 10 to 40% aqueous solution (manufactured by, for example, Tokyo Chemical Industry Co., Ltd.), and therefore, a reaction system according to the present invention absolutely contains water. However, TPAH is dissolved in DMSO or acetonitrile in a given ratio, and therefore the water content of the reaction solution is at most 20%. This is the reason why the dehydration process may be omitted.

Based on the above study, the application of a microreactor to the synthesis of [¹⁸F]SFB was studied.
As a first step, each of the steps of synthesis of [¹⁸F]SFB was performed using both a microreactor and a conventional reactor (macro-scale) to compare changes in yield with reaction time at different reaction temperatures.

### (First-Step Reaction and Second-Step Reaction)

A microchip shown in Fig. 3 was prepared as a microreactor used to perform the first-step reaction and the second-step reaction for the purposes of comparison with a macro-scale reaction. A microchip 1 is formed by bonding two glass substrates together. In the surface of one of the glass substrates, a reaction channel 3 and connection channels for connecting a reaction solution injection port, a reagent injection port, a liquid discharge port, and a reaction stop reagent injection port to the channel 3 are formed. In the other glass substrate, through holes are formed as a reaction solution injection port 7, a reagent injection port 9, a liquid discharge port 17, and a reaction stop reagent injection port 15. These two glass substrates are bonded together so that the reaction channel 3 and the connection channels are located inside. The channels and the through holes are formed by sandblasting, and the glass substrates are bonded by hydrofluoric acid bonding.

The reaction solution injection port 7 and the reagent injection port 9 are connected to one end 5 of the reaction channel 3 through their respective connection channels, and the liquid discharge port 17 and the reaction stop reagent injection port 15 are connected to the other end 3a of the channel 3 through their respective connection channels. The reaction channel 3 is a channel extending between the one end 5 and the other end 3a. Each of the reaction channel 3 and the connection channels has a width of 150 µm and a depth of 150 µm, and the reaction channel 3 has a length of 250 mm and a volumetric capacity of 5.625 µL

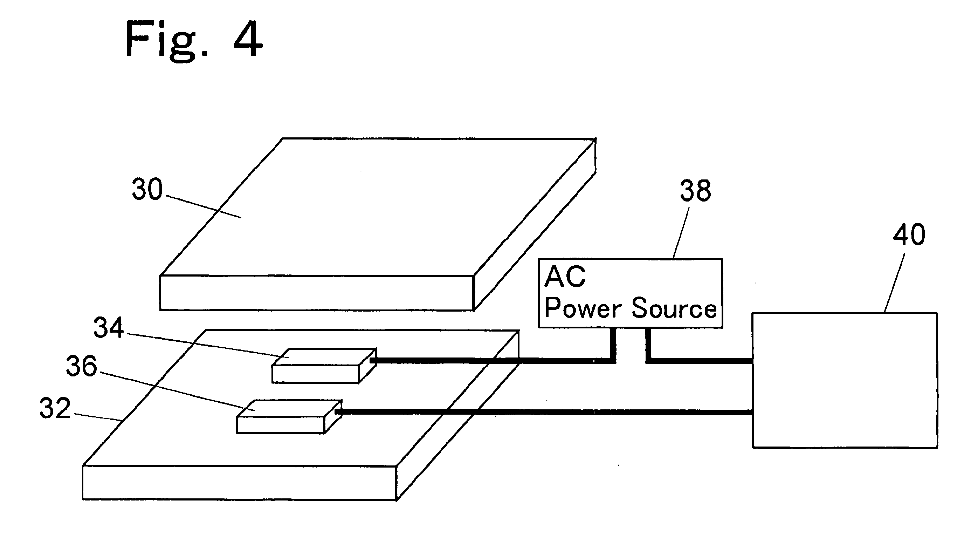

The microchip 1 was placed on an aluminum chip stage equipped with a built-in ceramic heater to perform a reaction at a predetermined temperature. One example of the chip stage is shown in Fig. 4. The chip stage includes two aluminum plates 30 and 32, a ceramic heater 34, and a temperature sensor 36 comprising a thermocouple, and the ceramic heater 34 and the temperature sensor 36 are interposed between the aluminum plates 30 and 32. The heater 34 is connected to a power source 38, and a temperature regulator 40 controls an electric current supplied from the power source 38 to the heater 34 based on a detection signal outputted from the temperature sensor 36 so that the plates 30 and 32 can have a predetermined temperature.

As the labeled precursor solution, an acetonitrile solution of the compound 1 (1 mg/mL) was used. As the first reagent, a K222/K[¹⁸F]F complex solution (10 mg/mL) was used. As the reagent used in the second-step reaction, a TPAH solution (1 M 20 µL/500 µL a solution of 500 µL containing 20 µL of a 1 M TPAH solution) was used. The first-step reaction was performed at both 90°C and 120°C, and the second-step reaction was performed at 120°C. These reactions were performed using both the conventional reactor (macro-scale) and the microreactor to compare a change in yield with reaction time. When the reaction temperature was 90°C, acetonitrile was used as a solvent, and when the reaction temperature was 120°C, DMSO having a high boiling point was used as a solvent

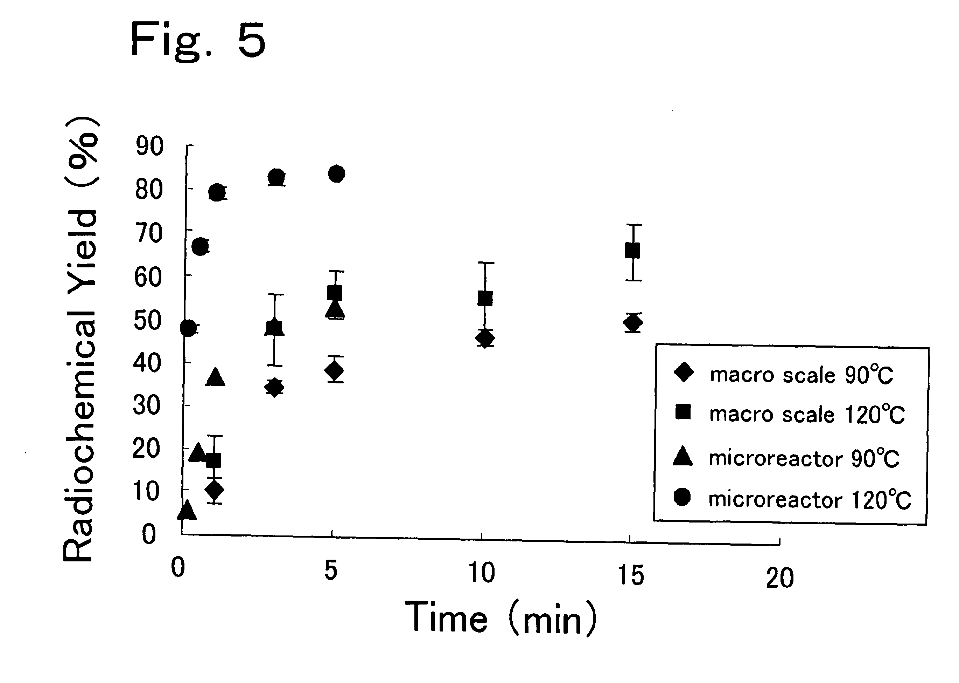

The results of the first-step reaction are shown in Fig. 5. In Fig. 5, the vertical axis represents a decay-corrected radiochemical yield. When the first-step reaction was performed using the microreactor, the yield was higher than when the first-step reaction was performed on a macro-scale, that is, a reduction in reaction time was achieved. This tendency became more conspicuous by increasing the reaction temperature from 90°C to 120°C.

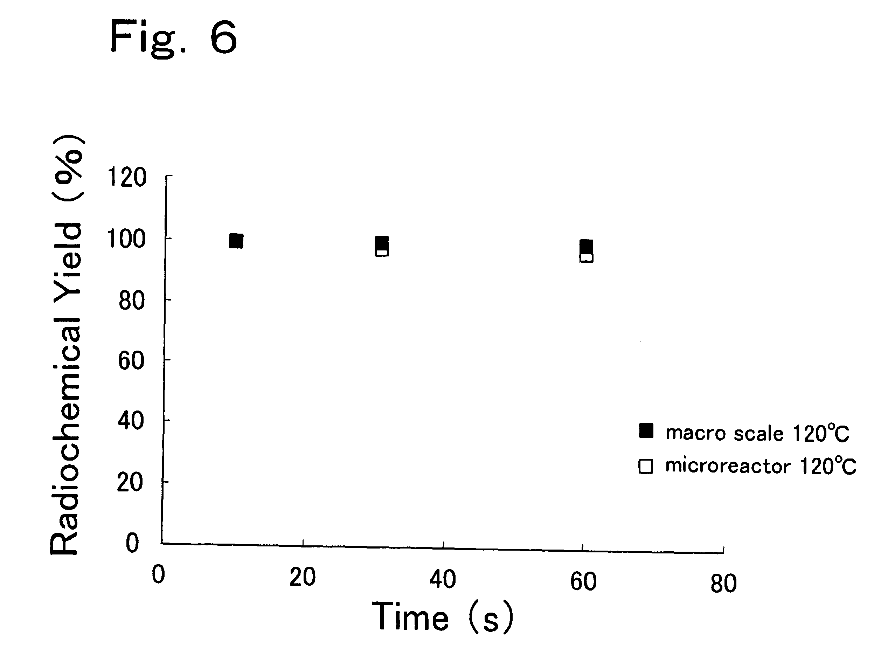

The second-step reaction was performed at 120°C using both the conventional reactor (macro-scale) and the microreactor. The results of the second-step reaction are shown in Fig. 6. In Fig. 6, the vertical axis represents a decay-corrected radiochemical yield. As can be seen from the results shown in Fig. 6, there is little difference between the macro-scale reaction and the reaction using the microreactor. It can be considered that the reason for this is that the second-step reaction rapidly occurs and therefore no difference occurs between the macro-scale reaction and the reaction using the microreactor under this reaction condition.

### (Study of Reaction Conditions)

As solvents, acetonitrile and DMSO were used. The reaction temperature and the type of solvent used were restudied by a macro-scale experimental system. This is because acetonitrile evaporates in the microreactor when the reaction temperature is 90 to 120°C. The reaction time of the first-step reaction was set to 10 minutes, the reaction time of the second-step reaction was set to 5 minutes, and the reaction time of the third-step reaction was set to 2 minutes. When acetonitrile was used as a solvent, three reaction temperatures of 70°C, 80°C, and 90°C were used, and when DMSO was used as a solvent, four reaction temperatures of 70°C, 80°C, 90°C, and 120°C were used. The three-step reaction was performed at these different temperatures. A comparison of the yield of [¹⁸F]SFB and the yield of the compound 2 (t-butyl 4-[¹⁸F]fluorobenzoate) was made by HPLC analysis.

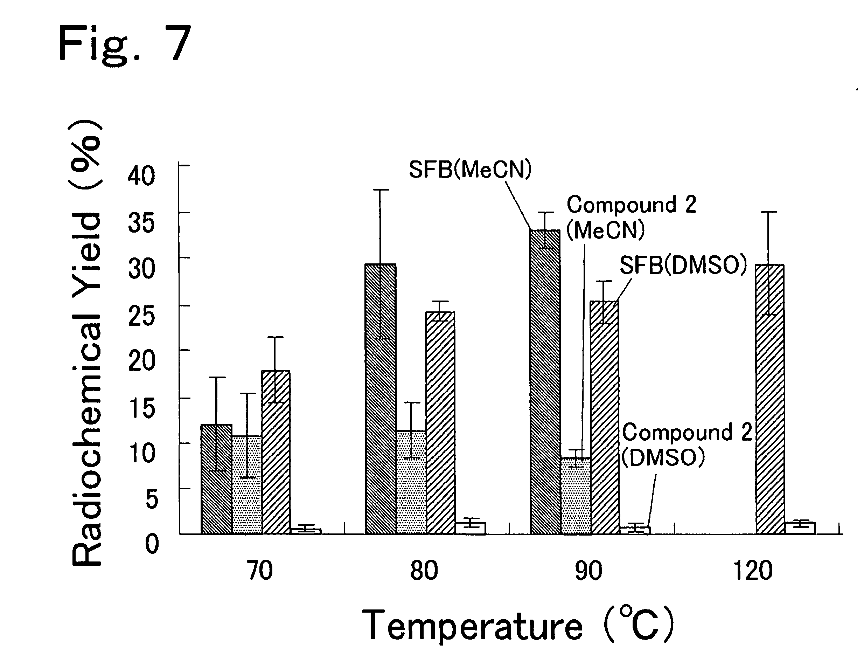

The results are shown in Fig. 7. In Fig. 7, the vertical axis represents a decay-corrected radiochemical yield. As can be seen from Fig. 7, an increase in the reaction temperature promotes the production of [¹⁸F]SFB and reduces the amount of the residual compound 2, and changing a solvent from acetonitrile to DMSO makes it possible to reduce the amount of the residual compound 2.

An optimum reaction time was determined based on the above results, and a one-flow synthesis of [¹⁸F]SFB was performed using a microchip for three-step reaction.

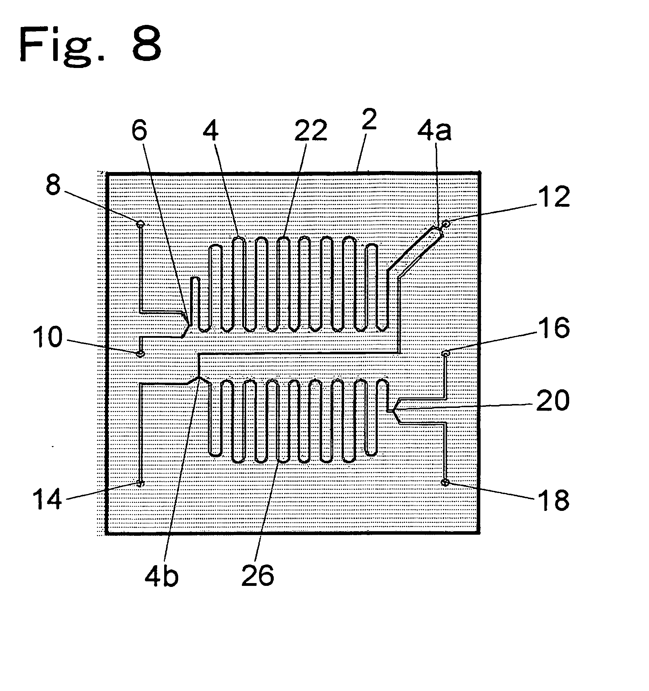

Fig. 8 shows a microchip as one example of a microreactor used to implement the present invention. A microchip 2 is formed by bonding two glass substrates together. In the surface of one of the glass substrates, a reaction channel 4 and connection channels for connecting a raw material injection port, a liquid discharge port, and injection ports for injecting various reagents to the channel 4 are formed. In the other glass substrate, through holes are formed as a raw material injection port 8, a liquid discharge port 18, and injection ports 10, 12, 14, and 16 for injecting various reagents. These two glass substrates are bonded together so that the reaction channel 4 and the connection channels are located inside. The channels and the through holes are formed by sandblasting, and the glass substrates are bonded by hydrofluoric acid bonding.

The raw material injection port 8 and the first reagent injection port 10 are connected to one end 6 of the reaction channel 4 through their respective connection channels, and the second reagent injection port 12 is connected to the channel 4 at a position far from the one end 6 of the channel 4, the third reagent injection port 14 is connected to the channel 4 at a position far from the second reagent injection port 12 in a direction toward the other end of the channel 4, and the liquid discharge port 18 is connected to the other end 20 of the channel 4. Further, the reaction stop reagent injection port 16 for injecting a reaction stop reagent is connected to the other end 20 of the channel 4 through its connection channel.

The reaction channel 4 includes a first-step reaction channel 22, a second-step reaction channel 24, and a third-step reaction channel 26. The first-step reaction channel 22 extends from the one end 6 to a position 4a at which the second reagent injection port 12 is connected to the channel 4, the second-step reaction channel 24 extends from the position 4a to a position 4b at which the third reagent injection port 14 is connected to the channel 4, and the third-step reaction channel 26 extends from the position 4b to the other end 20.

Each of the reaction channel 4 and the connection channels has a width of 150 µm and a depth of 150 µm. The first-step reaction channel 22 has a length of 250 mm and a volumetric capacity of 5.625 µL, the second-step reaction channel 24 has a length of 50 mm and a volumetric capacity of 1.125 µL, and the third-step reaction channel 26 has a length of 200 mm and a volumetric capacity of 4.5 µL. The second-step reaction rapidly proceeds, and therefore, the reaction channel 4 is designed so that the second-step reaction channel 24 has the shortest length.

The microchip 2 was placed on the chip stage shown in Fig. 4 to perform a reaction at a reaction temperature of 120°C.

A solution of the compound 1 (concentration: 1 mg/500 µL), a K222/K[¹⁸F]F complex solution (concentration: 10 mg/500 µL), a TPAH solution (1 M TPAH 40 µL/ 500 µL), and a TSTU solution (15 mg/500 µL) were prepared as the labeled precursor solution of [¹⁸F]SFB, the first reagent, the second reagent, and the third reagent, respectively, and were then introduced into the microchip through their respective injection ports at a flow rate of 0.56 µL/min, a flow rate of 0.56 µL/min, a flow rate of 1.12 µL/min, and a flow rate of 2.24 µL/min, respectively. As a solvent of these solutions, DMSO was used. The reaction time of the first-step reaction was 5 minutes, the reaction time of the second-step reaction was 30 seconds, and the reaction time of the third-step reaction was 1 minute.

In order to analyze a reaction product, DMSO/water (90/10) was introduced into the reaction stop reagent injection port 16 as a reaction stop reagent, and the resulting reaction solution was fractionated by HPLC and measured by a gamma counter to calculate a radiochemical yield. The HPLC used was reverse-phase HPLC, and a column used was COSMOSIL 5C₁₈-PAQ or COSMOSIL 5C₁₈-MSII (inner diameter. 4.6 mm, length: 150 mm, Nacalai Tesque). The HPLC was performed by gradient elution using a mixture of ammonium acetate buffer (50 mM, pH 4.0) (A) and MeCN (B) as a mobile phase. The volume ratio between A and B was set to 75A/25B at the beginning of HPLC, and was changed to be 20A/80B after a lapse of 20 minutes and 20A/80B after a lapse of 30 minutes. The flow rate of the mobile phase was 1 mL/min.

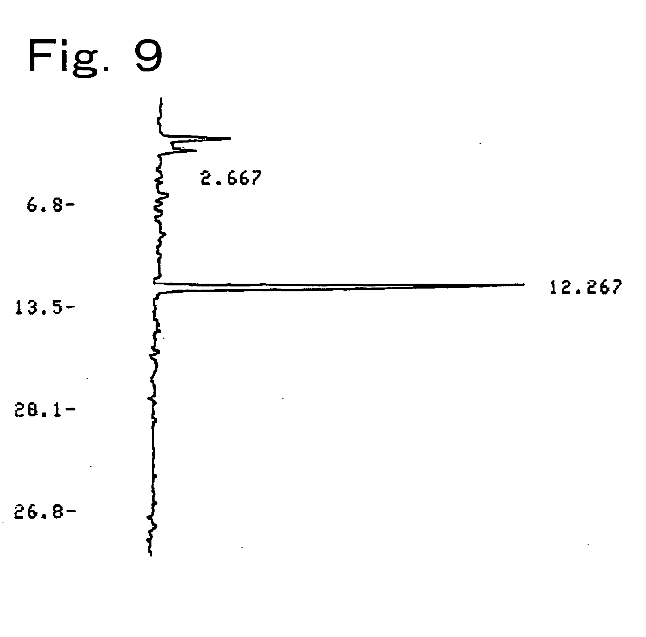

The liquid chromatogram of the reaction solution after reaction is shown in Fig. 9. The largest peak represents [¹⁸F]SFB. The radioactivity of ¹⁸F was measured by a Curie meter or a gamma scintillation counter. In Fig. 9, the horizontal axis represents time (min) and the vertical axis represents the number of counts.

The obtained result is shown in Table 1 as the result of Example. The results of conventional methods 1 to 4 are also shown in Table 1.

**Table 1**

| | Synthesis Time (min) | Yield (%) (decay-corrected) | Yield (%) (not decay-corrected) | References etc. |
|---|---|---|---|---|
| Example | 20 | 62.2 | 54.8 | The yields were calculated based on HPLC analysis. |
| Conventional Method 1 | 60 | 43.8 | 30.0 | J. Label. Compd. Radiopharm., 51, 68-71, 2008 |
| Conventional Method 2 | 78 | 46 | 28.1 | Appl. Radiat. Isot., 65, 199-203, 2007 |
| Conventional Method 3 | 68 | 36 | 23.4 | Appl. Radiat. Isot., 63, 329-332, 2005 |
| Conventional Method 4 | 120 | 49 | 23.0 | Nucl. Med. Biol., 31, 739-746, 2004 |

The conventional methods 1 to 4 are based on a one-pot procedure or a two-pot procedure and are disclosed in the above references.

The decay-corrected yield was calculated by the following equation: N = N₀/(1/2)^{(t/T)}, where N represents decay-corrected yield, No represents non-decay-corrected yield, T represents half-life, and t represents synthesis time.

As can be seen from the results shown in Table 1, according to the method of the present invention, [¹⁸F]SFB was obtained in a radiochemical yield (decay-corrected) of about 60% in a synthesis time of 20 minutes.

### Industrial Applicability

[¹⁸F]SFB synthesized by the method according to the present invention can be used to synthesize probes for use in PET diagnosis.

### Explanation of Reference Numerals

| | |
|---|---|
| 2 | microchip |
| 4 | reaction channel |
| 6 | one end of channel 4 |
| 8 | raw material injection port |
| 10 | first reagent injection port |
| 12 | second reagent injection port |
| 14 | third reagent injection port |
| 16 | reaction stop reagent injection port |
| 18 | liquid discharge port |
| 20 | another end of channel 4 |
| 22 | first-step reaction channel |
| 24 | second-step reaction channel |
| 26 | third-step reaction channel |

## Claims

1. A method for synthesizing [¹⁸F]SFB comprising:
using a microreactor including a substrate having one channel formed therein so as to have a cross-sectional width of 1 mm or less and a cross-sectional depth of 1 mm or less, the channel being connected to a raw material injection port and a first reagent injection port at one end thereof, and connected to a second reagent injection port at a position far from the one end thereof, and connected to a third reagent injection port at a position far from the second reagent injection port in a direction toward other end thereof, and connected to a liquid discharge port at the other end thereof; and
continuously performing the following three-step reaction by allowing a reaction solution to flow through the channel serving as a reaction channel from the one end to the other end thereof without taking out the reaction solution from anywhere between the one end and the other end of the channel:
a first-step reaction in which a solution of a labeled precursor of [¹⁸F]SFB and a solution containing ¹⁸F⁻ ions as a first reagent are continuously supplied from the raw material injection port and the first reagent injection port, respectively to fluorinate the labeled precursor in the channel between the one end and the second reagent injection port;
a second-step reaction in which a TPAH (tetrapropylammonium hydroxide) solution as a second reagent is continuously supplied from the second reagent injection port to saponify a reaction product of the first-step reaction in the channel between the second reagent injection port and the third reagent injection port so that a [¹⁸F]SFB intermediate is produced; and
a third-step reaction in which a TSTU
(O-(N-succinimidyl)-N,N,N',N' -tetramethyluronium tetrafluoroborate) solution as a third reagent is continuously supplied from the third reagent injection port to produce [¹⁸F]SFB in the channel between the third reagent injection port and the other end.

2. The method for synthesizing [¹⁸F]SFB according to claim 1, wherein acetonitrile is used as a solvent of the labeled precursor solution and the solution containing ¹⁸F⁻ ions to cause a reaction at a temperature lower than a boiling point of acetonitrile.

3. The method for synthesizing [¹⁸F]SFB according to claim 1, wherein DMSO (dimethyl sulfoxide) is used as a solvent of the labeled precursor solution and the solution containing ¹⁸F⁻ ions to cause a reaction at a temperature equal to or higher than a boiling point of acetonitrile but lower than a boiling point of DMSO.

## Patentansprüche

1. Verfahren zur Synthese von [¹⁸F]SFB, umfassend:
das Verwenden eines Mikroreaktors, einschließend ein Substrat mit einem darin gebildeten Kanal mit einer Querschnittsbreite von 1 mm oder weniger und
einer Querschnittstiefe von 1 mm oder weniger, wobei der Kanal an einem Ende mit einem Rohmaterialinjektionsanschluss und einem ersten Reagenzinjektionsanschluss verbunden ist und an einer Position, die weit von dem einen Ende entfernt ist, mit einem zweiten Reagenzinjektionsanschluss verbunden ist und an einer Position, die weit von dem zweiten Reagenzinjektionsanschluss in einer Richtung des anderen Endes entfernt ist, mit einem dritten Reagenzinjektionsanschluss verbunden ist und an dem anderen Ende mit einem Flüssigkeitsauslassanschluss verbunden ist; und
das kontinuierliche Durchführen der folgenden Reaktion mit drei Schritten durch das Fließenlassen einer Reaktionslösung durch den Kanal, der als Reaktionskanal dient, von dem einen Ende zu dem anderen Ende, ohne die Reaktionslösung von irgendwo zwischen dem einen Ende und dem anderen Ende des Kanals zu entnehmen:
ein erster Reaktionsschritt, in dem eine Lösung eines gelabelten Vorläufers von [¹⁸F]SFB und eine Lösung, enthaltend ¹⁸F-Ionen als ein erstes Reagenz, kontinuierlich jeweils von dem Rohmaterialinjektionsanschluss und dem ersten Reagenzinjektionsanschluss zugeführt werden, um den gelabelten Vorläufer in dem Kanal zwischen dem einen Ende und dem zweiten Reagenzinjektionsanschluss zu fluorieren;
ein zweiter Reaktionsschritt, in dem eine TPAH (Tetrapropylammonium-hydroxid)-Lösung als ein zweites Reagenz kontinuierlich von dem zweiten Reagenzinjektionsanschluss zugeführt wird, um ein Reaktionsprodukt des ersten Reaktionsschritts in dem Kanal zwischen dem zweiten Reagenzinjektionsanschluss und dem dritten Reagenzinjektionsanschluss zu verseifen, damit ein [¹⁸F]SFB-Intermediat entsteht; und
einen dritten Reaktionsschritt, in dem eine TSTU (O-(N-Succinimidyl)-N,N,N',N'-tetramethyluroniumtetrafluoroborat)-Lösung als ein drittes Reagenz kontinuierlich von dem dritten Reagenzinjektionsanschluss zugeführt wird, um [¹⁸F]SFB in dem Kanal zwischen dem dritten Reagenzinjektionsanschluss und dem anderen Ende herzustellen.

2. Verfahren zur Synthese von [¹⁸F]SFB nach Anspruch 1, worin Acetonitril als ein Lösemittel der Lösung des gelabelten Vorläufers und der Lösung, enthaltend ¹⁸F⁻-Ionen, verwendet wird, um eine Reaktion bei einer Temperatur, niedriger als ein Siedepunkt von Acetonitril, zu verursachen.

3. Verfahren zur Synthese von [¹⁸F]SFB nach Anspruch 1, worin DMSO (Dimethylsulfoxid) als ein Lösemittel der Lösung des gelabelten Vorläufers und der Lösung, enthaltend ¹⁸F⁻-Ionen, verwendet wird, um eine Reaktion bei einer Temperatur, gleich oder höher als ein Siedepunkt von Acetonitril, aber niedriger als ein Siedepunkt von DMSO, zu verursachen.

## Revendications

1. Une méthode pour synthétiser le [¹⁸F]SFB comprenant:
l'utilisation d'un microréacteur comprenant un substrat ayant un canal formé à l'intérieur de manière à avoir une largeur de section transversale de 1 mm ou
moins et une profondeur de section transversale de 1 mm ou moins, le canal étant connecté à une de ses extrémités à un port d'injection de matière première et à un port d'injection d'un premier réactif, et connecté à une position loin de cette extrémité à un port d'injection d'un deuxième réactif, et connecté à un port d'injection d'un troisième réactif à une position loin du port d'injection du deuxième réactif dans une direction située vers l'autre extrémité du canal, et
connecté à un port d'évacuation de liquide à l'autre extrémité du canal; et
effectuant continuellement la réaction trois étape suivante en permettant à une solution réactionnelle de s'écouler à travers la canal servant de canal de réaction d'une première extrémité vers l'autre extrémité sans enlever la solution réactionnelle, où que ce soit, entre la première extrémité et l'autre extrémité du canal :
une première étape réactionnelle dans laquelle une solution d'un précurseur de [¹⁸F]SFB marqué et une solution contenant les ions ¹⁸F⁻ comme premier réactif sont continuellement fournis par le port d'injection de matière première et le port d'injection du premier réactif respectivement pour fluorer le précurseur marqué dans le canal entre la première extrémité et le port d'injection du deuxième réactif ;
une deuxième étape réactionnelle dans laquelle une solution de HTPA (hydroxyde de tétrapropylammonium) comme deuxième réactif est continuellement fournie par le port d'injection du deuxième réactif pour saponifier un produit de réaction de la première étape réactionnelle dans le canal entre le port d'injection du deuxième réactif et le port d'injection du troisième réactif de sorte qu'un intermédiaire de [¹⁸F]SFB soit produit ; et
une troisième étape réactionnelle dans laquelle une solution de TSTU (tétrafluoroborate de O-(N-succinimidyl)-N,N,N',N'-tétraméthyluronium) comme troisième réactif est continuellement fournie par le port d'injection du troisième réactif pour produire le [¹⁸F]SFB dans le canal entre le port d'injection du troisième réactif et l'autre extrémité.

2. La méthode pour synthétiser le [¹⁸F]SFB selon la revendication 1, où l'acétonitrile est utilisé comme solvant de la solution de précurseur marqué et de la solution contenant les ions ¹⁸F⁻ pour engendrer une réaction à une température inférieure au point d'ébullition de l'acétonitrile.

3. La méthode pour synthétiser le [¹⁸F]SFB selon la revendication 1, où le DMSO (diméthylsulfoxyde) est utilisé comme solvant de la solution de précurseur marqué et de la solution contenant les ions 18F⁻ pour engendrer une réaction à une température supérieure ou égale au point d'ébullition de l'acétonitrile mais inférieure au point d'ébullition du DMSO.
